(19) Europäisches Patentamt
European Patent Office
Office européen des brevets

(11) Publication number : **0 393 104 B1**

(12) **EUROPEAN PATENT SPECIFICATION**

(45) Date of publication of patent specification : **15.07.92 Bulletin 92/29**

(51) Int. Cl.$^5$ : **G01N 33/53,** A61K 49/00

(21) Application number : **89900199.4**

(22) Date of filing : **30.11.88**

(86) International application number : **PCT/DK88/00197**

(87) International publication number : **WO 89/05452 15.06.89 Gazette 89/13**

(54) **MEASUREMENT OF PGE 2 (PROSTAGLANDIN E 2) LEVELS IN PATIENTS WITH CHRONIC INFLAMMATORY BOWEL DISEASE.**

(30) Priority : **01.12.87 US 127157**

(43) Date of publication of application : **24.10.90 Bulletin 90/43**

(45) Publication of the grant of the patent : **15.07.92 Bulletin 92/29**

(84) Designated Contracting States : **DE FR GB IT SE**

(56) References cited :
**DE-A- 3 015 793**
**Gastroenterology, vol. 95, no. 1, issued July 1988, K. Lauritsen, L. Laursen, K. Bukhave and J. Rask-Madsen, " In VivoProfiles of Eicosanoids un Ulcerative Colitis, Crohn s Coltis, and Clostridium Difficile Colitis."**
**Pharmacology & Toxicology, vol. 61, No. 4, issued October 1987, K. Lauritsen, L.Laursen, K.Bukhave and J.Rask-Madsen,"Does Vitamin E Supplementation Modulate in Vivo Arachidonate Metabolism in Human Inflammation?".**

(56) References cited :
**Gut, vol. 21, No. 7, issued July 1980, D.S.Rampton, G.E.Sladen and L.J.F.Youlten, "Rectal mucosal prostaglandin E2 releadeand its relation to disease activity, electrical potential difference, and treatment in ulcerative colitis."**

(73) Proprietor : **FONDEN FOR PROSTAGLANDINFORSKNING**
**Vilhelmshabsvej 10**
**DK-2920 Charlottenlund (DK)**

(72) Inventor : **RASK-MADSEN, Jorgen**
**Vilhelmshabsvej 10**
**DK-2930 Charlottenlund (DK)**
Inventor : **LAURITSEN, Karsten**
**Rypebakken 34**
**DK-5210 Odense NV (DK)**
Inventor : **BUKHAVE, Klaus**
**Hojdevej 8**
**DK-2830 Virum (DK)**

(74) Representative : **Mathiesen, Hans Preben et al**
**Chas. Hude 33 H.C. Andersens Boulevard**
**DK-1553 Copenhagen V (DK)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

### Technical Field

The present invention relates to a process (1) for evaluating disease activity in patients with chronic inflammatory bowel diseases (IBD), termed ulcerative colitis (UC) and Crohn's colitis (CC), respectively, and (2) for predicting (a) the outcome of medical treatment in severe, active disease, where acute or subacute surgery may be an alternative, in addition to (b) the risk of relapse in symptom-free patients, who wish to stop relapse preventing medication.

### Background Art

The antiinflammatory action of corticosteroids is well established and treatment with pharmacological doses of corticosteroids is the most important medical treatment in a large number of active inflammatory and immune based diseases. Thus corticosteroids are considered the most effective type of drugs for treatment of acute relapsing UC, but long-term treatment is associated with a number of serious side effects.

For long-term treatment of UC and CC in low-active phase it is more convenient, therefore, to use drugs containing 5-amino-salicylic acid (5-ASA) linked to sulphapyridine (sulphasalazine (SAZ), Salazopyrine™, Pharmacia AB, Sweden), another 'carrier' molecule (the recently developed sulphasalazine-analogues) or 5-ASA in a 'slow release' preparation, because free 5-ASA is completely absorbed in the upper small intestine. Thus the well-known adverse effects of corticosteroids are avoided and the risk of relapse markedly reduced.

Since the 1940's the most important 5-ASA containing preparation has been sulphasalazine (SAZ) with the chemical structure:

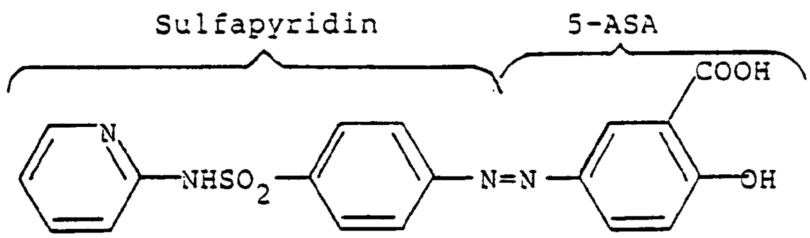

SAZ

SAZ, which passes the small intestine unabsorbed, is split in the colon by bacterial azo-reductases into its two components. Free 5-ASA acts directly on the colonic mucosa without prior absorption. Within the last few years a number of SAZ-analogous and 'slow-release' 5-ASA preparations have been developed for treatment of patients with IBD. In controlled clinical trials it has been attempted to decide whether patients with UC, refractory to SAZ-treatment, would benefit from treatment with the new sulphor-free salicylates, which may be administered in higher doses than SAZ without side effects. Although SAZ is an effective drug for maintenance treatment, with the purpose of avoiding a relapse of the disease, a large proportion of patients does not obtain a complete remission and relapses occur frequently during long-term maintenance treatment with SAZ. In addition, 10-20% of the patients are allergic to SAZ or in other ways intolerant of the drug. This is considered to be caused by sulphapyridine, which is easily absorbed when split from the therapeutically active moiety, 5-ASA. The efficacy of SAZ in preventing a relapse in patients with UC is dose dependent, but the frequency of adverse effects, which may be ascribed to sulphapyridine as mentioned above, increases with the dosage. Although oral administration of SAZ to patients with CC or mild to moderately active UC is effective the drug is markedly less effective than corticosteroids. By contrast, topical treatment with SAZ and 5-ASA appears to be as effective as topical treatment with glucocorticoids *i.e.* rectal instillation in patients with non-extensive disease localized to the lower colon or the rectum solely.

Lately, disodium-azodisalicylate (ADS; olsalazine: Dipentum™, Pharmacia AB, Sweden) has been appointed a promising candidate for all treatment of IBD, because the substance consists of two molecules of 5-ASA linked by an azo-bond. This means that each of its two active components acts as a carrier for the other:

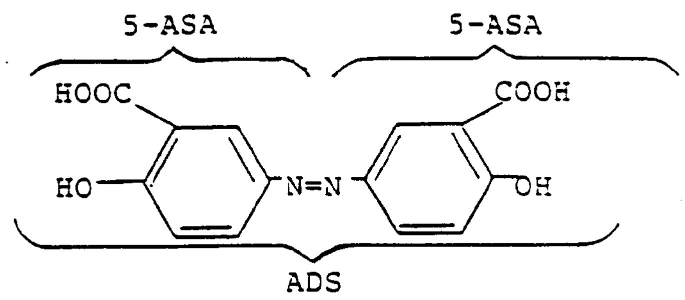

As previous reported by J.J. Misiewicz, J.E. Lennard-Jones, A. Connell, J.H. Baron, F.A. avery Jones, 'Controlled trial of sulphasalazine in maintenance therapy for ulcerative colitis', the Lancet 1965, 1:185-88, SAZ significantly reduces the relapse rate in patients with UC tolerant to the drug and is considered, therefore, the most important basic treatment for prevention of relapse in UC. However, it is still controversial whether SAZ treatment of symptom-free patients with UC should be continued more than one year and two early studies showed conflicting results. Although several later studies have shown a higher relapse rate in patients discontinuing SAZ treatment, the decision as regard duration of treatment is not based on objective measures, but rests solely on 'personal, clinical experience', since no clinical findings of conventional laboratory measurements improve decision making.

Furthermore, it is a serious problem that patients having had treatment for acute relapsing UC and subsequently maintained on SAZ to prevent a relapse often discontinue medication, thus increasing the risk of relapse and complications. Oppositely, it is inappropriate to continue treatment which is not justified for medical reasons.

## Description of the Invention

It is therefore an object of the invention to provide a process enabling assessment of disease activity in patients with active UC and CC and prediction of the outcome of medical treatment in severe disease, where surgery may be the alternative, said process also enabling judgement of the risk of relapse in patients with clinically inactive UC and CC duting maintenance therapy.

In order to satisfy the above objects and advantages provided by the present invention a process for (1) assessment of disease activity in patients with chronic inflammatory bowel disease characterized as ulcerative colitis (UC) and Crohn's colitis (CC) and for (2) prediction of (a) efficacy of medical treatment in case of active disease prior to start of therapy and (b) risk of relapsing disease in case of clinically inactive disease in association with or following discontinuation of relapse preventing medical treatment wherein equilibrium in vivo dialysis of faeces and/or equilibrium in vivo dialysis of rectum is carried out for determination of faecal and/or rectal concentrations of prostaglandin $E_2$ ($PGE_2$). The equilibrium dialysis ensures a preliminary purification of luminal $PGE_2$, which makes specific and accurate analysis possible.

Thus in connection with the present invention it has been shown that the concentration of prostaglandin (PG) $E_2$ in the rectal lumen is a highly sensitive marker of disease activity and that pretreatment $PGE_2$ concentrations are significantly higher in patients not responding to therapy than in those responding to therapy. Furthermore, it has been demonstrated herein that an increase in the luminal concentrations of $PGE_2$ in patients with inactive UC precedes the development of symptoms, in addition to objective clinical findings, abnormal histology of the colonic mucosa, and changes in conventional laboratory indices in patients who later experience a relapse.

Using an advantageous embodiment of the process of the invention the determination of faecal concentrations of $PGE_2$ is carried out on the pooled contents of orally administered dialysis bags to eliminate possible variations due to differences in the transit time of bags having passed the gastrointestinal tract and determination of rectal concentrations of $PGE_2$ is carried out on the contents of a dialysis bag which has been placed for four hours in the patient's emptied rectum.

Using this embodiment of the process of the invention concentrations of $PGE_2$ can be determined radioimmunologically by a method validated by gas chromatography-mass spectrometry and quantitative high pressure liquid chromatography using 10-100 μl dialysate, $^3H$-labelled $PGE_2$ as an internal standard, acidification, extraction with ethylacetate/cyclohexane (1:1) and chromatography on microcolumns of Sephadex™ LH-20 before performing the radioimmunoassay itself on the eluate fraction containing the purified $PGE_2$. Other methods of determining the concentration of $PGE_2$ could also be used, e.g., an ELISA or other immunologic method.

The test kit to be used in the process of the invention is characterized by including dialysis bags for oral

intake and/or rectal insertion, in addition to essential and specific test substances for the performance of radioimmunological determination of $PGE_2$ in the contents of the dialysis bags.

According to the invention the dialysis bags are preferably manufactured from Visking seamless tubing 8/32 (with an average pore size of 2,4 nm) by tying off 3 or 12 cm segments with a suture ("Poly" from Ethicon) and filling the segments (i.e. the dialysis bags) with Rheomacrodex™ 100 mg/l (i.e. 100 g dextran 40 and 9 g saline per liter).

According to the invention the test substances contained in the kit are lyophilized antibodies against $PGE_2$, tritium labelled $PGE_2$, and non-radiolabelled $PGE_2$.

Brief Description of the Drawings

Fig. 1 illustrates how the concentrations of $PGE_2$ in the rectal lumen of patients with ulcerative colitis are positively correlated to clinical, endoscopic, as well as histologic disease activity.

Fig. 2 illustrates how the concentrations of $PGE_2$ in the rectal lumen of 13 untreated patients with relapsing ulcerative colitis change after treatment with Pentasa (5-aminosalicylic acid) enemas (1 g/day) for 2-4 weeks and the individual outcome of treatment is classified as clinical and endoscopic remission, clinical but not endoscopic remission, and unchanged or worse.

Fig. 3 illustrates how the concentrations of $PGE_2$ in the rectal lumen of 11 untreated patiens with relapsing ulcerative colitis change after treatment with prednisolone enemas (25 mg/day) for 2-4 weeks and the individual outcome of treatment is classified as clinical and endoscopic remission, clinical but not endoscopic remission, and unchanged or worse.

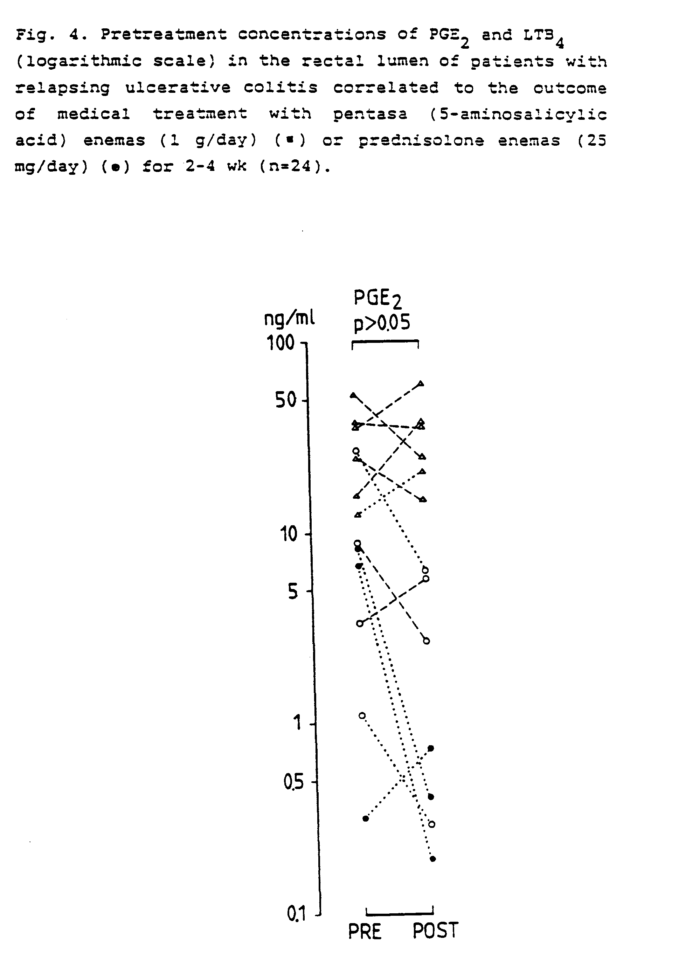

Fig. 4 illustrates how pretreatment concentrations of $PGE_2$ in the rectal lumen of 24 patients with relapsing ulcerative colitis correlate to the outcome of medical treatment with pentasa (5-aminosalicylic acid) enemas (1 g/day) or prednisolone enemas (25 mg/day) for 2-4 weeks. The results show that pretreatment concentrations of $PGE_2$ may be used as a predictor of the outcome of medical treatment with the named drugs.

Best Mode for carrying out the Invention

**Material and Methods (I)**

PATIENTS AND CONTROLS

The study group comprised the first 24 patients from the Department of Medical Gastroenterology, Odense University Hospital, entering a randomized, double-blind Danish multicenter trial for comparison of the therapeutic efficacy of topical 5-ASA and prednisolone in ulcerative proctosigmoiditis. The inclusion criteria were as follows: (a) proven UC (V. Binder, H. Both, P.K. Hansen, C. Hendriksen, S. Kreiner, K. Torp-Pedersen, 'Incidence and prevalence of ulcerative colitis and Crohn's disease in the county of Copenhagen', Gastroenterology 1982,83:563-8) localized to the sigmoid colon or the rectum, or both; (b) symptoms and signs of mild ($C_1$) or moderate ($C_2$) disease activity (V. Binder, 'A comparison between clinical state, macroscopic and microscopic appearances of rectal mucosa, and cytologic picture of mucosal exudate in ulcerative colitis', Scandinavian Journal of Gastroenterology 1970,5:627-32); (c) no drug treatment for UC the preceding month apart from maintenance treatment with sulphasalazine (SAZ); (d) outpatient capable of administering enemas following instruction; (e) normal renal and hepatic function. For patients maintained on SAZ (1 g b.i.d.) this treatment was kept unchanged throughout the trial. No other drugs were given.

In 8 healthy volunteers (3 men, 5 women; aged 18-69 yr) serving as controls, equilibrium in vivo dialysis of rectum was carried out on a single occasion.

The study was done in accordance with the Helsinki Declaration II and approved by the Ethical Committee of Funen and vejle Counties. Informed consent was given by all participants.

EXPERIMENTAL DESIGN

The study was carried out as a block-randomized, double-blind trial. Before entry, clinical disease activity was assessed, and equilibrium in vivo dialysis of rectum was performed before the assessment of endoscopic and histologic disease activity. The patients were randomly allocated to one of the two treatments, *i.e.*, 100 ml enemas containing either 1000 mg 5-ASA in acidic buffer suspension, pH 4.8 (Pentasa; Ferring A/S, Copenhagen, Denmark) or 25 mg prednisolone (Predniment, Ferring), which were given once daily at bedtime for 14 days. Primary stratification was done for patients already on maintenance treatment with SAZ. At day 15 the equilibrium *in vivo* dialysis of rectum was repeated, and clinical, endoscopic, and histologic activities were reassessed. Patients in complete clinical and endoscopic remission discontinued treatment with enemas, whereas the remainder continued therapy for another 14 days. In all cases equilibrium *in vivo* dialysis of rectum and assessment of disease activity were repeated at day 29. Withdrawal criteria included intolerance to treatment or deterioration (severe disease, $C_3$) requiring systemic glucocorticoids.

Laboratory screening was performed on all study days and included blood hemoglobin, reticulocyte count, erythrocyte sedimentation rate, leukocyte count, leukocyte differential count, platelet count, and serum concentrations of creatinine and orosomucoid, in addition to urine analysis for protein and microscopy for erythrocytes, leukocytes, and casts.

DISEASE ACTIVITY

The disease activity was assessed at each study day according to a four-scaled semiquantitative grading: (a) *clinical activity*: inactive ($C_o$), mild ($C_1$), moderate ($C_2$), severe ($C_3$); (b) *endoscopic activity* inactive ($E_o$), mild ($E_1$), moderate ($E_2$), severe ($E_3$); (c) *histologic activity*: inactive ($H_o$), mild ($H_1$), moderate ($H_2$), severe ($H_3$).

The clinical activity was based on a diary in which the number of bowel movements and the presence or absence of blood were registered by the patient and the objective findings were registered by the investigator. To minimize variation among observers each patient was examined on all study days by the same investigator, who was unaware of the results of the rectal dialyses.

EQUILIBRIUM IN VIVO DIALYSIS OF RECTUM

In vivo dialysis of rectum was performed after insertion of a 12-cm-long dialysis bag (volume 4 ml; Visking seamless cellulose tubing 8/32 filled with Rheomacrodex®; Pharmacia, Uppsala, Sweden) containing 10% dextran (mean mol wt 40,000) in saline into the emptied rectum. To obtain equilibrium with the fluid covering the surface of rectal mucosa, the dialysis bag was left for 4 h before its contents were emptied into a test tube, which was stored immediately at -20°C (rectal dialysate) for analysis within 2 wk.

To ensure that $PGE_2$ had reached an equilibrium, intact dialysis bags were incubated with ($^3$H)$PGE_2$ (50,000 cpm/ml) in phosphate-buffered saline (pH 7.4; 37°C). After 1, 3, and 4 h, respectively, the radioactivities within the bags were 50%, 80%, and 85% of the measured in the external solution. Binding of $PGE_2$ to the dialysis membrane was negligible (K. Lauritsen, J. Hansen, P. Bytzer, K. Bukhave, J. Rask-Madsen, 'Effects of sulphasalazine and disodium azodisalicylate on colonic $PGE_2$ concentrations determined by equilibrium in vivo dialysis of faeces in patients with ulcerative colitis and healthy controls', Gut 1984,25:1271-8).

ANALYTIC PROCEDURES

Prostaglandin $E_2$ was measured as previously described in detail (K. Bukhave, J. Rask-Madsen J, 'Prostaglandin $E_2$ in jejunal fluids and its potential diagnostic value for selecting patients with indomethacin-sensitive diarrhoea', 1981 European Journal of Clinical Investigation, 11:191-7) by a radioimmunologic method validated by gas chromatography-mass spectrometry (K. Bukhave, K Gréen, J. Rask-Madsen, 'Comparison of radioimmunological determinations with gas chromatography mass spectrometry dosage', 1983 Biomedical Mass Spectrometry, 10:265-8). The method included purification by extraction with ethylacetate/cyclo- hexane (1:1) and chromatography on microcolumns of Sephadex LH-20 (Pharmacia AB, Sweden) before performing the radioimmunoassay itself on the eluate fraction containing $PGE_2$.

STATISTICAL ANALYSES

Nonparametric statistics were used. The results were given as medians with ranges and $Q_{50}$ interquartile ranges (defined by the lower and upper quartiles). The data were analyzed by the Chi square method, the Mann-Whitney test for unpaired variates, Wilcoxon's test for paired variates, or Kendall's rank correlation test as indicated either in the text or in footnotes in the tables. Values of $p<0.05$ were considered significant.

**Results (I)**

DESCRIPTION AND COMPARISON OF TREATMENT GROUPS

The patients consecutively entered the trial and equilibrium in vivo dialysis of rectum was done three times in all cases but one. The condition of the latter patient, who was on 5-ASA, deteriorated and this patient was consequently withdrawn after the second week of treatment to receive systemic glucocorticoids. Table 1 describes patient characteristics and the outcome of treatment in the two groups. There was no statistically significant difference with regard to age, sex, duration of disease, present disease activity, number of patients treated with SAZ, or outcome of the enema treatments with or without adjuvant SAZ.

In retrospect, the pretreatment assessment of clinical disease activity was a poor predictor of the outcome of treatment ($p>0.05$) in contrast to endoscopic and histologic indices (Table 2).

One patient in each treatment group complained of nausea, and 2 patients on 5-ASA reported difficulties in retaining the enemas. The laboratory screening disclosed no clinically significant abnormalities, except for a slight increase in platelet counts and serum concentrations of orosomucoid in a few cases.

$PGE_2$ CONCENTRATIONS IN THE RECTAL LUMEN OF HEALTHY VO LUNTEERS AND PATIENTS WITH UC

*Healthy volunteers.* Concentrations of $PGE_2$ in rectal lumen of healthy controls (n=8) ranged from 0.15 to 0.59 ng/ml (median 0.32 ng/ml).

*Patients with active ulcerative colitis.* The luminal concentrations of $PGE_2$ were markedly elevated compared to healthy controls ($p<0.05$, Mann-Whitney test). A significant positive correlation ($p<0.05$, Kendall's rank correlation test) was found between concentrations of $PGE_2$ and disease activity judged by clinical, endoscopic, or histologic gradings (fig. 1). Also, some patients with clinically or endoscopically inactive disease ($C_o$ or $E_o$) had increased $PGE_2$ levels compared to healthy controls (Table 3). In the rectal dialysates obtained from patients assessed to have the same grade of disease activity, no statistically significant differences ($p>0.05$) were observed (a) between levels of $PGE_2$ in patients allocated to 5-ASA and prednisolone enemas, respectively, (b) between levels in patients untreated or off treatment and levels in patients treated with enemas, and (c) between levels in patients with or without adjuvant SAZ treatment (Table 3). However, the numbers of observations within some of these subgroups were small.

EFFECTS OF 5-AMINOSALICYLIC ACID AND PREDNISOLONE ON RECTAL PROSTAGLANDIN $E_2$ CONCENTRATIONS IN ULCERATIVE COLITIS

Fig. 2 shows the effect of treatment with 5-ASA enemas on $PGE_2$ concentrations in the rectal lumen of patients with active UC. No significant effects on the named variables could be traced in the whole group ($p>0.5$, Wilcoxon's test for paired variates), but $PGE_2$ levels decreased toward control levels in those patients responding to treatment.

Similarly, fig. 3 illustrates the results of treatment with prednisolone enemas, which inhibited $PGE_2$ concentrations significantly ($p<0.05$).

RELATIONSHIP BETWEEN PRETREATMENT PROSTAGLANDIN $E_2$ CONCENTRATIONS IN ULCERATIVE COLITIS AND OUTCOME OF TREATMENT

Fig. 4 illustrates that the pretreatment $PGE_2$ levels were signifcantly higher ($p<0.05$, Mann-Whitney test) in the patients not responding to therapy than in those who improved. The laboratory screening was normal apart from marginally elevated serum concentrations of orosomucoid in some of the former patients, all of whom required systemic glucocorticoids to control disease activity.

**Discussion (I)**

Since S.R. Gould, 'Prostaglandins, ulcerative colitis, and sulphasalazine' 1975 The Lancet, 2:988, first reported raised concentrations of PG-like material in stools from patients with an acute attack of UC, many in vitro studies have shown that the amount of $PGE_2$ and the activities of PG synthetase measured in fresh biopsy specimens of rectal mucosa (as well as the generation of $PGE_2$ and the stable breakdown products of $PGI_2$ and thromboxane $A_2$ in cultures of rectal mucosa from patients with relapsing UC) were significantly higher than those obtained in material from healthy controls. These observations have been confirmed in vivo by means of the method of equilibrium dialysis of faeces (K. Lauritsen, J. Hansen, P. Bytzer, K. Bukhave, J. Rask-Madsen, 'Effects of sulphasalazine and disodium azodisalicylate on colonie $PGE_2$ concentrations determined by in vivo dialysis of faeces in patients with ulcerative colitis and healthy controls', 1984 Gut, 25:1271-8). In spite of these publications, it has not been previously appreciated that the level $PGE_2$ in a patient could be a valid marker of disease activity as well as a useful predictor of the outcome of medical treatment and of the risk of relapse. The method of equilibrium in vivo dialysis of rectum used in the present study permitted the demonstration of a close positive correlation between $PGE_2$ and disease activity. This observation supports the notion

that arachidonic acid metabolism plays a pathophysiologic role in UC. The PGs enhance vasodilation and edema formation, acting synergistically with other mediators of inflammation (*e.g.*, histamine, bradykinin), whereas the leukotrienes promote leukocyte migration and stimulate aggregation and degranulation of neutrophils, in addition to release of lysozomal enzymes and superoxide production. A major argument against the hypothesis that the role of $PGE_2$ is primary rather than secondary is the observation that treatment with potent cyclooxygenase inhibitors, such as indomethacin and flurbiprofen, has no beneficial clinical effect, and may even provoke a relapse, in the face of reduced levels of PGs in the urine and in the rectum. However, the named compounds do not affect the production of 5-lipoxygenase metabolites. On the other hand, drugs with proven efficacy in UC, such as the glucocorticoids and SAZ, seem to share such an effect, at least in vitro: The glucocorticoids are considered to inhibit phospholipase activity, necessary for the liberation of arachidonic acid from membrane bound phospholipids, thereby leading to decreased metabolism along both cyclooxygenase and lipoxygenase pathways. Similarly, 5-ASA, the active moiety of SAZ, inhibits the lipoxygenase system in human neutrophils. The results of the present study, which demonstrate that rectal concentrations of $PGE_2$ decrease toward control levels in patients who respond to therapy (*i.e.*, 5-ASA or prednisolone), may, however, be interpreted to reflect the mode of drug action. Furthermore, the reduction in rectal $PGE_2$ levels seems to occur promptly (*i.e.*, within 12-72 h), at least after high-dose systemic treatment with glucocorticoids (K. Lauritsen, L. S. Laursen, K. Bukhave, J. Rask-Mad sen, 'In vivo effects of orally administered prednisolone on prostaglandin and leucotriene production in ulcerative colitis', 1987 Gut, 28:1095-99).

The patients who entered this study were only part of a larger multicenter clinical trial for comparison of the therapeutic efficacy of the two drug regimens. In this connection, it should be noted that most patients with high pretreatment $PGE_2$ concentrations in the rectum were treatment failures and required high-dose systemic glucocorticoids to control the disease. Approximately 15% of all patients with UC confined to the rectum and sigmoid colon are resistant to most forms of therapy, and the management of so-called refractory proctosigmoiditis an unrewarding task. If pretreatment levels of $PGE_2$ reflect local disease activity, they may prove more useful predictors of the outcome of medical treatment in patients with UC than clinical indices of disease activity.

**Material and Methods (II)**

PATIENTS

Patients fulfilling the following entry criteria were candidates for the study: (a) proven UC or proven CC; (b) relapsing disease (*i.e.*, a relapse within the past six months) or chronic continous disease activity (*i.e.* absence of remission (definition: see below) for three months or more); (c) refractory to SAZ (*i.e.*, relapsing disease despite maintenance treatment with SAZ 2 g/day or chronic continous disease despite treatment with glucocorticoids and SAZ) or intolerant of SAZ (*i.e.*, allergic to or otherwise intolerant of SAZ).

Patients below 18 or above 80 years of age were excluded, as were women who were pregnant or lactating and those with childbearing potential not using oral contraception or an intrauterine device. Furthermore, patients with CC, who had also radiologic verified small bowel disease were excluded.

Each patient gave informed consent and the study was approved by the Ethical Committee of Funen and Vejle Counties.

EXPERIMENTAL DESIGN

The study design was open with an initial dose of ADS 1 g/day (0.5 g *b.i.d.*), in SAZ intolerant patients. In SAZ refractory patients the initial dose was 2 g/day (1 g *b.i.d.*). The dose was gradually increased at the scheduled visits until the endpoint was reached (*i.e.*, sustained remission - as defined below), or drug intolerance occurred, or a maximum of 4 g/day (1 g *q.i.d.*) equivalent to approximately 10 g/day of SAZ as regards 5-ASA on a molar basis. In patients on concomitant corticosteroids the dose was gradually tapered and, if possible, the administration was stopped.

A one year treatment period was planned including scheduled visits at two weeks and at two, four, six, eight, ten, and 12 months, in addition to in-between visits, if needed. Before entry and at each visit the clinical disease activity was assessed (see below) and a laboratory screening was done. This included blood haemoglobin, reticulocyte count, erythrocyte sedimentation rate, leucocyte count, leucocyte differential count, platelet count, serum concentrations of Na, K, Ca, albumin, creatinine, urea, alanine aminotransferase, lactate dehydrogenase, alkaline phosphatase, bilirubin, and plasma prothrombin, in addition to urine analysis for protein and glucose and microscopy for erythrocytes, leukocytes, and casts. Additional serum was obtained for determination of ADS, 5-aminosalicylic acid (5-ASA), and acetyl-5-aminosalicylic acid (Ac-5-ASA).

Before entry, at the two week's visit, and at the six and 12 months' visits equilibrium *in vivo* dialysis of faeces

as well as equilibrium *in vivo* dialysis of rectum were done prior to endoscopy of rectum and sigmoid colon with biopsies. ADS was administered in gelatin capsules, each containing 250 mg of the powdered drug without additives.

Compliance to prescription was estimated at each study visit by counting the number returned ADS capsules.

DISEASE ACTIVITY

The disease activity was graded according to the fourscaled semiquantitative grading: (a) clinical activity: inactive ($C_0$), mild ($C_1$), moderate ($C_2$), severe ($C_3$); (b) endoscopic activity: inactive ($E_0$), mild ($E_1$), moderate ($E_2$), severe ($E_3$); and (c) histologic activity: inactive ($H_0$), mild ($H_1$), moderate ($H_2$), severe ($H_3$). The clinical activity was based on a diary and the objective findings registered by the investigator. To minimize variation among observers each patient was examined on all study days by the same investigator. All days the week preceeding a scheduled visit the patient recorded the number of bowel movements in the diary, in addition to the presence or absence of blood, and graded abdominal pain and general well-being according to W. R. Best, J. M. Becktel, J. W. Singleton, 'Rederived values of the eight coefficients of the Crohn's Disease Activity Index (CDAI)', 1979 Gastroenterology, 77:843-6) for calculation of the Crohn's disease activity index (CDAI). Remission in patients with UC was defined as clinically inactive disease ($C_0$; *i.e.*, below three bowel movements a day without blood present) and no evidence of inflammation ($E_0$) on sigmoidoscopy. Remission in patients with CC was defined as a CDAI <150 and no evidence of inflammation on sigmoidoscopy. Patients in whom remission did not occur without concomitant use of corticosteroids were classified as treatment failures. Due to the open design of the study no further clinical or endoscopic estimations of efficacy were done.

*IN VIVO* DIALYSIS METHODS

Equilibrium *in vivo* dialysis of faeces was done as previously described in detail by pooling the contents of five swallowed dialysis bags following their intestinal transit (faecal dialysate). These dialysates were analyzed for concentrations of ADS, 5-ASA, and Ac-5-ASA, in addition to $PGE_2$ (faecal $PGE_2$).

Equilibrium *in vivo* dialysis of rectum was performed after insertion of a 12-cm-long dialysis bag into the emptied rectum as previously described (K. Lauritsen, L.S. Laursen, K. Bukhave, J. Rask-Madsen, "Effects of topical 5-aminosalicylic acid and prednisolone on prostaglandin $E_2$ and leukotriene $B_4$ levels determined by equilibrium in vivo dialysis of rectum in relapsing ulcerative colitis, Gastroenterology 1986;91:837-844). The bag was left for four hours and its contents (rectal dialysate) were analyzed for concentrations of $PGE_2$ (rectal $PGE_2$).

ANALYTICAL PROCEDURES

Determination of ADS, 5-ASA, and Ac-5-ASA in serum and faecal dialysates were done by high performance liquid chromatography (HPLC) as described above. The lower detection limits in serum and in faecal dialysates were 1.0 umol/l and 6.0 umol/l, respectively. Prostaglandin $E_2$ was measured by the radioimmunologic method, referred to above, validated by gas chromatography-mass spectrometry. These measurements were done blindly and independently of the assessment of disease activity and clinicians were unaware of the results until the data had been completed.

REFLECTIONS ON SAMPLE SIZE

This trial was a pilot series with the purpose of providing information for the planning of controlled trials of long term ADS treatment in UC and CC. Among all it was speculated that in such trials any fixed-dose regimen might be associated wits changed pharmacokinetical characteristics (*e.g.*, incomplete splitting of the azo bond and subsequently changed luminal concentrations of 5-ASA in active disease) depending on the extension and the severity of disease, which might influence the tolerance and the efficacy of the drug. It was considered relevant to compare colonic olsalazine metabolism and tolerance, (a) in patients with extensively located colitis and is patients with 'left-sided' colitis, (b) in SAZ refractory patients and is SAZ intolerant patients, (c) in patients with chronic continuously active disease and in patients with intermittently active disease, (d) in patients with severe disease activity ($C_3$) and in patients with moderate and mild disease activity ($C_1$-$C_2$), and (e) in patients with UC and CC. For each such comparison a group size of eight to 12 was considered to provide relevant pharmacologic information. It was estimated, therefore, that inclusion of a total of 40 to 50 patients in the study would be desirable.

STATISTICAL ANALYSES

The changes, if any, in safety variables after entry were tested for trends using the paired t-test and the Wilcoxon matched pairs signed ranks test, as appropriate. Values of $p<0.05$ were considered significant.

**Results (II)**

DESCRIPTION OF PATIENTS

Forty patients (31 with UC and nine with CC) consecutively entered the study within a six month period. Four more patients with UC and one with CC fulfilled the entry criteria within the period of accrual but refused participation. Table 4 describes patient characteristics. The duration of treatment and the mean dose per patient and total doses used are shown in Table 5, whereas Table 6 gives the duration of the individual dosages administered throughout the treatment period.

WITHDRAWALS, TOLERANCE, AND SAFETY

Eight patients did not complete the planned one year treatment period: two patients with UC and one with CC had a colectomy performed due to lack of efficacy after four, seven, and eight months, respectively. One patient with a 12 year history of UC had a colectomy after six months' treatment because biopsies revealed a rectal mucosal adenocarcinoma. Three patients wished to stop treatment prematurely, in two cases because of lack of efficacy (one with UC after eight months, one with CC after ten months), and a third with CC after three months because she was in complete remission. Finally, one patient with UC in remission was excluded after eight months because he had not taken the study medicine.

Tolerance problems after ADS prescription such as loose or watery stools and/or increased stool frequency were reported in 11 cases (seven with UC, four with CC). Intolerance occurred most often (nine cases) in patients with extensively located disease and active disease (nine cases), but was transient in eight cases (*i.e.*, resolved within a few weeks during continuous administration of the drug). No patients were withdrawn due to drug intolerance. Two patients reported transient pain in the bones and joints, respectively. The Wilcoxon matched pairs signed ranks test revealed no significant differences in laboratory screening values before and after the trial. In six cases slightly increased levels of alanine aminotransferase or alkaline phosphatase were found, but the values normalized during continuous treatment.

CONCENTRATIONS OF OLSALAZINE, 5-AMINOSALICYLIC ACID, AND ACETYL-5-AMINOSALICYLIC ACID IN FaecAL DIALYSATES

The average concentrations of ADS, 5-ASA, Ac-5-ASA, and total 5-ASA (5-ASA + Ac-5-ASA) measured in faecal dialysates from each patient were calculated for each dosage of ADS. The results obtained in patients with UC are given in Table 7. The results obtained in the nine patients with CC were within the range of those obtained in UC treated with equal dosages of ADS, but the number of observations in the CC group precluded statistical comparison with the UC group. Concentrations of Ac-5-ASA in faecal dialysates were rather constant within the applied ADS dose range (from 1 g/day to 4 g/day), but concentrations of free 5-ASA, and thus also concentrations of total 5-ASA increased dose dependently (Table 7). ADS levels in faecal dialysates were low (Table 7) compared to those of the split product (5-ASA) and its metabolite (Ac-5-ASA). The efficacy of azoreduction was estimated by calculation of the 'split ratio', *i.e.*, the ratio between molar concentrations in faecal dialysates of total 5-ASA and total 5-ASA + 5-ASA contents in unsplit ADS, respectively. The median split ratio was 99.4% ($Q_{50}$ interquartile range 93.8-100%, range 40.1-100%) in all dialysates obtained from patients with UC. In 28% of all patients the split ratio was below 90%, at least on one occasion, and more than half (six of 11) of these cases reported transient intolerance to the prescribed ADS dose. Resolution of drug intolerance was associated with an increased split ratio.

During active disease the mean concentrations of 5-ASA and Ac-5-ASA in faecal dialysates tended to be lower than in dialysates from patients in remission (Table 8), but the number of observations on each dose of ADS was too small to permit statistical analysis. No differences between 5-ASA levels in patients with extensively located colitis and patients with 'left-sided' disease were found.

CONCENTRATIONS OF OLSALAZINE, 5-AMINOSALICYLIC ACID, AND ACETYL-5-AMINOSALICYLIC ACID IN SERUM

The average serum concentrations of ADS, 5-ASA, and Ac-5-ASA in each patient were calculated for each dosage of ADS. The results are given in Table 9 for patients with UC. Dose dependent increases in the levels of olsalazine and 5-ASA were observed within the whole dose range, but no further increase in serum concentrations of Ac-5-ASA occurred beyond a dose of ADS equal to 3 g/day (Table 9). No statistically significant differences ($p>0.05$) were found between serum concentrations of ADS, 5-ASA, and Ac-5-ASA in patients with UC and CC, respectively, when treated with an equal dosage of ADS. Similarly, no statistically significant differences ($p>0.05$) were found between levels of ADS in patients with active disease and patients in remission (Table 8) or in patients with pancolitis and patients with 'left-sided' disease. In individual patients on a stable dose of ADS no increase in serum concentrations of the unsplit drug or its metabolites were observed in the long term.

EFFECTS ON PROSTAGLANDIN $E_2$ IN FaecAL AND RECTAL DIALYSATES

The levels of $PGE_2$ in faecal and rectal dialysates decreased significantly over time in patients with UC (Table 10). Concentrations of $PGE_2$ were significantly raised in patients with active UC ($C_1$-$C_3$) compared to those obtained in patients in remission ($C_0$). In 13 UC patients, in whom remission never occurred (treatment failures), pre-trial rectal $PGE_2$ concentrations were significantly raised ($p<0.05$) compared to those observed in patients not classified as treatment failures. The rectal $PGE_2$ concentrations in treatment failures persisted markedly increased during the medication period, whereas those observed in the remaining cases decreased toward the normal range. Thus also post-trial rectal $PGE_2$ concentrations were increased in treatment failures ($p<0.05$). All but one of seven patients with pre-treatment rectal $PGE_2$ concentrations >30 ng/ml were classified as treatment failures.

By contrast, no statistically significant trends were observed in $PGE_2$ levels in patients with CC (Table 10).

ESTIMATES ON EFFICACY

Thirteen patients with UC were regarded as treatment failures (*i.e.*, clinical and sigmoidoscopic remission was never obtained). This outcome was observed in one of eight patients intolerant of SAZ and in 12 of 23 patients refractory to SAZ ($p=0.12$, Fisher's exact test). In six cases the disease was 'left-sided', whereas seven cases had a pancolitis. In the remaining patients a relapse occurred on at least one occasion: four experienced a relapse on 1 g/day, two on 2 g/day, eight on 3 g/day, and two on 4 g/day.

Five of the nine patients with CC were classified as treatment failures (*i.e.*, a CDAI <150, without sigmoidoscopic evidence of inflammation, was never obtained).

**Discussion (II)**

Previous studies have shown that small intestinal absorption and metabolism of ADS is minimal and that a single oral dose is completely recovered from ileostomy fluid (H. Sandberg-Gertzén, M. Ryde, G. Järnerot, 'Absorption and excretion of a single 1-g dose of Azodisal sodium in subjects with ileostomy' 1983 Scandinavian Journal of Gastroenterology, 18:107-11). In patients with inactive UC complete azoreduction of olsalazine occurs on 1 g *b.i.d.* and concentrations of 5-ASA in faecal dialysates, in fact, double when SAZ is replaced by the same dose of ADS (K. Lauritsen, J. Hansen, M. Ryde, J. Rask-Madsen, 'Colonic azodisalicylate metabolism determined by *in vivo* dialysis in healthy volunteers and patients with ulcerative colitis', 1984 Gastroenterology, 86:1496-1500). Oral ADS 0.5 g *b.i.d.* appears more effective than placebo in preventing relapse in SAZ intolerant patients (H. Sandberg-Gertzén, G. Järnerot, W. Kraaz, 'Azodisal sodium in the treatment of ulcerative colitis. A study of tolerance and relapse-prevention properties' , 1986 Gastroenterology, 90:1024-30) and 1 g *b.i.d.* orally for two weeks is more effective than placebo in the treatment of mildly active UC (W. S. Selby, G. D. Barr, A. Ireland, C. H. Mason, D. P. Jewell, 'Olsalazine in active ulcerative colitis', 1985 Br Med J, 291:1373-5). The major adverse effect in these preliminary clinical trials was loose or watery stools, which were observed only in 10% of patients. This complaint usually occurred in patients with extensively located colitis and might be explained by the action of the unsplit ADS molecule that, unlike SAZ, markedly increases ileostomy output

(H. Sandberg-Gertzén, G. Järnerot, K. Bukhave, K. Lauritsen, J. Rask-Madsen, 'Effect of azodisal sodium and sulphasalazine on ileostomy output of fluid, $PGE_2$, and $PGF_{2\alpha}$ in subjects with a permanent ileostomy', 1986 Gut,27:1306-11). The efficacy of azoreduction in active disease is unknown, but indirect evidence points

to a reduced azoreduction of SAZ. Among all it might be speculated that the intestinal transit time as well as the character of intestinal microflora depend on the extension and the severity of the disease and thus the distribution of ADS and its metabolites. This concept is supported by the present results demonstrating that intolerance is associated with insufficient azoreduction. The increased incidence of diarrhea observed in the present series most likely reflects the use of a higher dose of ADS than in the above mentioned previous study. Symptoms of this type of intolerance call, therefore, for a temporary reduction of the dose and the problem is more readily handled in clinical practice than in controlled trials with a fixed-dose regimen. Also the occurrence of diarrhea, which often subsided spontaneously with continued therapy, suggests that the therapeutic effect of 5-ASA may influence colonic function to permit a more effective azoreduction.

The present results also demonstrate that high doses of ADS can be administered safely in the long term and that azoreduction of ADS is almost complete in the major proportion of patients, even during administration of the highest dose (4 g/day). In this pharmaceutical respect, the ADS capsules fulfils the anticipation of providing a highly effective means of delivery of 5-ASA to the colonic mucosa, even in active disease. The results accord with our previous experience in patients with inactive UC and in healthy controls. No other 'second generation' candidate reported on and developed to replace SAZ - whether azobound or a pharmaceutically modified free 5-ASA preparation - has hitherto been shown to provide colonic concentrations of 5-ASA in the same order of magnitude as those demonstrated in the present study.

The findings of rather constant concentrations of Ac-5-ASA in faecal dialysates and dose dependently increasing concentrations of 5-ASA, indicate that saturation of the intraluminal acetylation capacity occurs at a low dosage. This may be clinically important, because a therapeutic effect of Ac-5-ASA has not been unequivocally established (C. P. Willoughby, J. Piris, S. C. Truelove, 'The effect of topical N-acetyl-5-aminosalicylic acid in ulcerative colitis', 1980 Scand J Gastroenterol,15:715-9 and V. Binder, S. Halskov, E. Hvidberg, et al., 'A controlled study of 5-acet-aminosalicylic acid (5-Ac-ASA) as enema in ulcerative colitis' (Abstract) 1981 Scand J Gastroenterol,16:1122). The serum concentrations observed on ADS 1 g/day and 2 g/day are comparable to those reported in healthy controls. The results (Table 9) indicate a dose related increase in absorption of ADS and its metabolites, but do not suggest that cumulation occurs in the long term.

The demonstration of extremely high intraluminal levels of $PGE_2$ in treatment failures accords with our previous findings and suggests that these quantifications of local disease activity are useful predictors of the outcome. We propose such determinations should be used prospectively in clinical trials on drug efficacy in UC.

The design of the present study did not permit any estimation of a dose response effect or a correlation between faecal 5-ASA levels and response to treatment. In planning the study we chose for safety reasons the pragmatic viewpoint to include only 'problematic' patients, who were not satisfactorily managed by conventional treatment (*i.e.*, SAZ and a short course of glucocorticoid), although ADS may prove to be a SAZ alternative. More treatment failures occurred among patients refractory to SAZ than among patients intolerant of SAZ. The complete splitting of olsalazine and the high concentrations of free 5-ASA in faecal dialysates from SAZ refractory patients, who did not experience a remission, and from patients who relapsed, although maintained on a 4-g dose of ADS, indicate that SAZ resistance is not merely a question of dosage, at least in a range up to 10 g/day (*i.e.*, equal to 4 g/day of ADS).

In conclusion, prolonged administration of high doses of ADS to patients with UC and CC appears acceptably well tolerated. The drug is a very effective means of delivery of 5-ASA to the colonic mucosa and, despite some cases of therapeutic resistance to even high intraluminal concentrations of 5-ASA, ADS seems to be a drug particularly appropriate for testing the efficacy of 5-ASA through controlled trials in patients with UC and CC in the long term.

## Material and Methods (III)

PATIENTS

Patients fulfilling the following entry criteria were candidates for the study: (a) proven UC. The diagnosis had been established on the basis of symptoms, endoscopic findings, histology on the rectal mucosa, and radiologic appearance, in addition to an exclusion of infectious diarrhea by stool cultures and microscopy; (b) inactive disease (remission) for at least 12 mo during maintenance treatment with sulphasalazine (SAZ; Pharmacia AB, Uppsala, Sweden) 2 g/day. Patients were considered in remission when symptom-free (i.e., stool frequency 2 x a day, without discharge of blood, pus, or mucus from the rectum), with normal sigmoidoscopic appearance, and with no significant inflammation on rectal biopsy.

The study was done in accordance with the Helsinki Declaration II and approved by the Ethical Committee of Funen and Vejle Counties. Informed consent was given by all participants.

## EXPERIMENTAL DESIGN

Equilibrium *in vivo* dialysis of faeces as well as equilibrium *in vivo* dialysis of rectum were done in consenting patients fulfilling the entry criteria. Subsequently, SAZ treatment was stopped and the patients were observed free of medication. Evaluation of clinical disease activity, in addition to sigmoidoscopy with rectal biopsies for assessing endoscopic and histologic disease activity, were done at scheduled visits after two weeks, after two, six, and 12 months, and in-between, if indicated by recurrence of colitic symptoms. A relapse was defined as a recurrence of colitic symptoms accompanied by endoscopic signs of inflammation. If a relapse occurred the patient was withdrawn. At the scheduled visits also *in vivo* dialysis measurements were carried out. If faecal PGE concentrations and/or rectal $PGE_2$ concentrations exceeded control levels (i.e., faecal $PGE_2$ >0.5 ng/ml; rectal $PGE_2$ >1.0 ng/ml) at any study day the patient was allocated at random to double blind maintenance treatment with SAZ (tablets 0.5 g; 2 tablets *b.i.d.*) or placebo tablets (2 tablets *b.i.d.*) of identical appearance for six months. The SAZ and placebo tablets were provided by Pharmacia AB, Uppsala, Sweden. The randomisation was done according to a computer generated list in blocks of four, stratified for visit (two weeks, two months, six months, 12 months). In randomized patients a final visit was scheduled after six months of randomisation for *in vivo* dialysis measurements and for assessment of clinical, endoscopic, and histologic disease activity in case a relapse had not occurred earlier.

To minimize the variation between observers each patient was examined on all study days by the same investigator, who was unaware of the results of the dialysis measurements.

A laboratory screening was performed on all study days and included blood haemoglobin, sedimentation rate, leucocyte count, leucocyte differential count, platelet count, and serum concentrations of creatinine and orosomucoid.

Thus patients completed the trial either (a) after 12 months of passive observation (in cases with normal $PGE_2$ levels throughout); or (b) six months after randomisation (in case $PGE_2$ levels exceeded control levels); or (c) in-between, if a relapse occurred.

Results obtained in the cohort of randomized patients served to evaluate the primary purpose of the study, that is, to test the predictive value of 'an increase in $PGE_2$ levels' for experiencing a relapse in patients with inactive UC off SAZ treatment. Furthermore, this cohort also permitted a secondary purpose of evaluating the efficacy of SAZ in maintaining remission in the long term.

## IN VIVO DIALYSIS METHODS

Equilibrium *in vivo* dialysis of faeces was carried out as previously described, by pooling the contents of five swallowed dialysis bags following their intestinal transit (faecal dialysate). These dialysates were analyzed for concentrations of $PGE_2$ (faecal $PGE_2$).

Equilibrium *in vivo* dialysis of rectum was performed after insertion of a 12-cm-long dialysis bag into the emptied rectum as described above. The bag was left for four hours when it was removed and its contents (rectal dialysate) were analyzed for concentrations of $PGE_2$ (rectal $PGE_2$).

## ANALYTICAL PROCEDURES

Prostaglandin $E_2$ was measured as previously described by a radioimmunological method validated by gas chromatograghy mass spectrometry. The method, using 10-100 ul samples, included addition of $^3$H-labelled $PGE_2$ (Amersham International, Buckinghamshire, U.K.) as an internal standard, acidification, extraction with ethylacetate/cyclohexane (1:1), and chromatograghy on microcolumns of Seghadex® LH-20 (Pharmacia AB, Uppsala, Sweden) before performing the radioimmunoassay itself on the eluate fraction. The detection limit, defined as the procedure blank value +2 SD, was 0.03 ng/ml.

Further evaluations of the radioimmunological procedures for determination of $PGE_2$ in 'rectal dialysates' were performed by comparing data obtained by simultaneous analysis of aliquots of the same samples (obtained from patients with active UC and controls) by radioimmunoassay and quantitative high pressure liquid chromatograghy for $PGE_2$. All organic solvents were from Merck, Darmstadt, F.R.G. The results were expressed as ng/ml of colonic fluid. In ten healthy controls the following results have previously been obtained: Faecal $PGE_2$ <0.5 ng/ml and rectal $PGE_2$ <0.6 ng/ml.

## STATISTICAL ANALYSIS

Fisher's exact test was used for analysis of fourfold tables. A value of $p < 0.05$ was considered significant.

**Results**

DESCRIPTION OF PATIENTS

Twenty-four patients consecutively entered the trial within an 18-month period. One patient was withdrawn because revision of her records showed that she had CC. Three more patients fulfilled the entry criteria, but refused participation. Table 11 describes characteristics of 23 patients eligible for the study.

PROSTAGLANDIN $E_2$ LEVELS IN FaecAL AND RECTAL DIALYSATES

Table 12 lists the faecal and rectal $PGE_2$ levels in individual patients throughout the trial. In 11 patients faecal $PGE_2$ levels exceeded 0.5 ng/ml (six cases) or rectal $PGE_2$ levels exceeded 1.0 ng/ml (all 11 cases) after withdrawal of SAZ: in six cases at two weeks; in one case at two months; in two cases at six months; and in two cases at 12 months (Table 12). Six of these patients were randomized to SAZ and five were randomized to placebo. The outcome of the disease within the six-month treatment period is also shown in Table 12: In the SAZ group none of the six patients experienced a relapse and $PGE_2$ concentrations decreased toward control levels. By contrast, four of five patients in the placebo group experienced a relapse ($p < 0.05$, Fisher's exact test).

Twelve patients remained non-randomized and ten of these experienced a relapse. Retrospectively: In two cases (both relapsing within two weeks after cessation of SAZ treatment) $PGE_2$ levels were increased at entry (Table 12: no. 1 and 3); in four cases (Table 12: no. 6, 9, 11, and 13) the $PGE_2$ levels increased more than two fold, but did not reach the randomisation levels. The $PGE_2$ concentrations persisted within control levels in the patients not randomized and not experiencing a relapse (Table 12: no. 7 and 21).

CLINICAL OUTCOME

The clinical outcome is listed in Table 12. All relapses were proven by histology of rectal biopsies. As described above, a relapse occurred in none of six and in four of five patients, respectively, randomized to SAZ and placebo ($p=0.03$) and in ten of 12 non-randomized patients. The cumulative relapse rate among patients not randomized to SAZ (*i.e.*, cumulative numbers with a relapse/numbers at risk off SAZ) was 53% (10/19) and 78% (14/18) after six and 12 months, respectively (Table 12).

In all patients relapsing, and thus withdrawn from the trial, remission was reinstated: in seven cases by resuming SAZ treatment 2-3 g/day orally; in six cases by resuming SAZ 2-3 g/day orally, in addition to topical treatment with corticosteroids, for two to eight weeks; and in a single case after prolonged treatment with systemic corticosteroid, in addition to SAZ.

**Discussion (III)**

The present results suggest that increased $PGE_2$ concentrations in rectal dialysis fluid determined by equilibrium *in vivo* dialysis of rectum are predictive of a relapse in patients with inactive UC. First, in the cohort randomized (because such an increase in $PGE_2$ concentrations had occurred) four of five patients allocated to placebo relapsed within six months, whereas the other six patients, allocated to SAZ, all remained in remission and showed a subsequent decrease in $PGE_2$ concentrations toward control levels. Second, in both patients, who relapsed within 14 days after cessation of SAZ therapy, the $PGE_2$ concentrations proved to have exceeded control levels at entry. Third, in none of the ten remaining patients the demonstration of a normal $PGE_2$ value was followed by an immediate (*i.e.*, within two weeks) relapse. However, the observation, that only two of these patients completed the observation period of 12 mo in remission, demonstrates that the 'lack of an increase in $PGE_2$ concentration' is predictive for not experiencing a relapse in the short term only. Thus, to improve decision-making and to limit the risk of a relapse in patients off SAZ treatment would require more frequent dialysis collections than performed in the present study.

The faecal and rectal $PGE_2$ concentrations determined by the methods of equilibrium *in vivo* dialysis of faeces and equilibrium *in vivo* dialysis of rectum, respectively, are both sensitive markers of disease activity. In patients with inactive UC an increased $PGE_2$ concentration in luminal dialysis fluid, whether faecal or rectal, seems indicative of a mucosal abnormality, even in the absence of conventional signs of inflammation. The data indicate that the rectal dialysis method is more sensitive than the *in vivo* dialysis of faeces: in all but one case (Table 12) the detection of an increased faecal $PGE_2$ concentration (>0.5 ng/ml) was associated with a concomitant increase in the rectal $PGE_2$ concentration (>1.0 ng/ml) and in no case a patient was randomized because of an increased faecal (but a normal rectal) $PGE_2$ concentration. Oppositely, an increased rectal $PGE_2$

concentration was often accompanied by a normal faecal $PGE_2$ concentration (Table 12). This may be of clinical relevance, because outpatients experience the collection of stools more tedious than the performance of equilibrium *in vivo* dialysis of rectum.

The cumulative relapse rates observed among patients off SAZ therapy in the present study are in keeping with previous results, which have shown a relapse rate of 55% and 69% over six months, and 75% over 12 months. Furthermore, the present data are in agreement with the findings of A. S. Dissanayake, S. C. Truelove, 'A controlled therapeutic trial of long-term maintenance treatment of ulcerative colitis with sulphasalazine (Salazopyrin)',1973 Gut,14:923-6 and later confirmed else-where, that SAZ is superior to placebo or other remedies, such as a high-fiber diet or mast-cell stabilizers, in maintaining remission in UC in the long term.

Based on the present data it might be argued that all patients with inactive disease should stay on SAZ indefinitely, because the relapse rate was high. However, the recurrences experienced in the present series were easily handled in most cases and the courses were uncomplicated. To decide on cessation or continuation of SAZ in patients with inactive UC, one needs, therefore, to address other aspects of this relapse preventing intervention, for example, potential prognostic gains, side effects, costs, in addition to patients' own expectations and wishes. The latter is sometimes expressed as a keen desire to discontinue the longstanding drug intake. The present study describes a method for improving decision-making in individual patients demanding to go off SAZ. The data demonstrate that an increased $PGE_2$ concentration in rectal dialysis fluid identify patients with a substantial risk of relapsing. However, only a few experience prolonged remission, which is the reason why frequent measurements are warranted.

Table 1 *Description of groups and outcome of treatment*

| | 5-Aminosalicylic acid (n=13) | Prednisolone (n=11) |
|---|---|---|
| Age, mean year (range)[a] | 27 (18-55) | 38 (24-66) |
| Sex, male/female[b] | 7/6 | 4/7 |
| Duration of disease, median yr (range) | 3 (0.5-15) | 4.5 (0.5-20) |
| Clinical activity, mild ($C_1$/moderate ($C_2$)[b] | 3/10 | 5/6 |
| Endoscopic activity, mild ($E_1$)/moderate ($E_2$/ severe ($E_3$)[b] | 1/6/6 | 2/8/1 |
| Histologic activity[c] mild ($H_1$)/moderate ($H_2$/ severe ($H_3$)[b] | 1/4/6 | 2/4/2 |
| Additional maintenance treatment, with/without sulfasalazine (2g/day)[b] | 7/6 | 6/5 |
| Duration of treatment, 2 wk/4 wk[b] | 6/7 | 4/7 |
| Outcome of treatment[b] | | |
| Clinical and endoscopic remission (on SAZ) | 3 (1) | 8 (3) |
| Clinical but not endoscopic remission (on SAZ) | 4 (3) | 1 (1) |
| Unchanged or worse (on sulfasalazine) | 6 (3) | 2 (2) |

[a]Not significant ($p>0.05$) by Mann-Whitney test. [b]Not significant ($p>0.05$) by Chi square method. [c]Biopsy specimens were insufficient for histologic grading in 5 patients.

Table 2 nt gradings of clinical, endoscopic ogic disease activity as predictors of treatment with 5-aminosalicylic acid olone enemas for 2 - 4 weeks.

| Pretr semiqua gra | Outcome of treatment mprovement (n=16) | Unchanged or worse (n=8) | Probability value[a] |
|---|---|---|---|
| Clinica | | | $p > 0.05$ |
| $C_1$ | 6 | 2 | |
| $C_2$ | 10 | 6 | |
| Endosco | | | $p < 0.01$ |
| $E_1$ | 3 | 0 | |
| $E_2$ | 12 | 2 | |
| $E_3$ | 1 | 6 | |
| Histolc | | | $p > 0.05$ |
| $H_1$ | 3 | 0 | |
| $H_2$ | 7 | 1 | |
| $H_3$ | 2 | 6 | |

[a]Chi sq histolc [b]Biopsy specimens were insufficient for in 5 patients.

Table 3 Concentrations of $PGE_2$ (ng/ml) in rectal lumen of patients with ulcerative colitis at different grades of activity, untreated or treated with 5-aminosalicylic acid or prednisolone enemas and/or sulfasalazine

| | | | | | | | | |
|---|---|---|---|---|---|---|---|---|
| 5-ASA | +/- | 0.71(n=7) (0.18-6.0) | 24(n=5) (2.5-58) | 33(n=8) (14-120) | 0.75(n=3) (0.18-3.7) | 5.5(n=4) (0.29-6.2) | 24(n=8) (2.5-58) | 31(n=5) (20-120) |
| Predni-solone | +/- | 1.6(n=9) (0.16-5.0) | 5.4(n=6) (0.58-42) | 9.5(n=3) (5.9-12) | 0.82(n=8) (0.16-5.0) | 2.8(n=7) (0.57-3.9) | 10.5(n=3) (5.5-42) | - - |
| None[d] | +/- | 0.62(n=6) (0.47-2.2) | 9.8(n=9) (0.35-36) | 9.0(n=18) (0.32-52) | 0.61(n=5) (0.47-2.2) | 3.2(n=5) (0.35-8.8) | 8.2(n=17) (0.32-36) | 21(n=6) (8.9-5.2) |
| | + | 0.61(n=3) (0.47-2.2) | 9.4(n=5) (0.35-36) | 8.5(n=9) (0.32-52) | 0.61(n=3) (0.47-22) | 3.2(n=3) (0.35-5.6) | 8.2(n=8) (0.32-36) | 36(n=3) (8.9-52) |
| | - | 0.62(n=3) (0.52-1.2) | 9.3(n=4) (3.4-11) | 10(n=9) (3.4-26) | 0.86(n=2) (0.52-1.2) | 4.7(n=2) (0.62-8.8) | 8.2(n=9) (3.4-26) | 16(n=3) (11-26) |

5-ASA, 5-aminosalicylic acid; $PGE_2$, prostaglandin $E_2$. Values represent median (range). [a]SAZ, sulfasalazine: whole groups (+/-) irrespective of SAZ therapy or not, or subgroups with (+) or without (-) SAZ maintenance therapy. [b]Clinical activity: 0 (inactive), 1 (mild), 2-3 (moderate-severe). [c]Endoscopic activity: 0 (inactive), 1 (mild), 2 (moderate), 3 (severe). [d]None, before enema treatment or off treatment.

Table 4 *Description of patients.*

| | Ulcerative colitis (n=31) | Crohn's colitis (n=9) |
|---|---|---|
| Age, mean year (range) | 34(19-66) | 28(18-34) |
| Sex, male/female | 16/15 | 2/7 |
| Smokers/non-smokers | 3/28 | 7/2 |
| Duration of disease, mean year (range) | 6.7(1.8-19) | 5.8(3.0-10) |
| Course last year, continuously active/intermittently active | 20/11 | 7/2 |
| Sulphasalazine, refractory/intolerant | 23/8 | 8/1 |
| Extension of disease, extensively located ('total')/'left sided' | 12/19 | 9/0 |

Table 5 *Duration of treatment and olsalazine doses used in patients with ulcerative colitis and Crohn's colitis*

| | | Ulcerative colitis (n=31) | Crohn's colitis (n=9) |
|---|---|---|---|
| Duration of treatment | days | 341±69 | 319±95 |
| | | (132-398) | (93-383) |
| Mean daily dose[a] | g/day | 2.6±0.7 | 2.5 ±0.9 |
| | | (1.0-3.6) | (1.0-3.5) |
| Total dose in treatment period | g | 872±310 | 794±396 |
| | | (369-1349) | (217-1309) |

Values represent mean±SD (range). [a]Calculated as the ratio between total dose in treatment period and days of treatment.

Table 6 *Duration of individual olsalazine dosages administered in 31 patients with ulcerative colitis and 9 patients with Crohn's colitis*

| | | Olsalazine dose/day | | | |
|---|---|---|---|---|---|
| | | 1 g | 2 g | 3 g | 4 g |
| Ulcerative colitis | Numbers on dose | 12 | 29 | 21 | 22 |
| | Days on dose | 165±147 | 116±107 | 99±50 | 133±68 |
| | | (14-395) | (14-381) | (32-194) | (49-244) |
| Crohn's colitis | Numbers on dose | 3 | 8 | 5 | 4 |
| | Days on dose | 218±160 | 102±119 | 133±108 | 172±73 |
| | | (39-349) | (14-371) | (31-295) | (78-257) |

Values of 'days on dose' represent mean ±SD (range).

Table 7 *Concentrations of olsalazine, 5-ASA, and Ac-5-ASA in faecal dialysates of patients with ulcerative colitis: averages in each patient for each dosage of olsalazine*

| | | Olsalazine dose/day | | | |
|---|---|---|---|---|---|
| | | 1 g (n=7) | 2 g (n=20) | 3 g (n=14) | 4 g (n=18) |
| Olsalazine | mmol/l | 0.10±0.14 (<0.006-0.72) | 0.34±0.48 (<0.006-1.7) | 0.40±0.58 (<0.006-2.0) | 0.27±0.41 (<0.006-1.4) |
| 5-ASA | mmol/l | 5.7±4.9 (<1.0-18) | 8.0±6.1 (<1.0-31) | 13±11 (<1.0-37) | 23±12 (6.7-55) |
| Ac-5-ASA | mmol/l | 11±8.5 (<0.7-26) | 9.5±9.0 (<0.7-60) | 7.5±8.2 (<0.7-26) | 11±5.6 (2.0-26) |
| Total 5-ASA | mmol/l | 17±9.0 (<1.7-40) | 18±14 (<1.7-72) | 20±18 (<1.7-63) | 34±17 (8.6-75) |

5-ASA = 5-aminosalicylic acid; Ac-5-ASA = acetyl-5-aminosalicylic acid. Values represent means±SD (range)

Table 8 *Mean concentrations of olsalazine in serum and 5-ASA and Ac-5-ASA in fecal dialysates in patients with active ($C_1$-$C_3$) versus inactive ($C_0$) ulcerative colitis on different doses of olsalazine*

| | Disease activity | Olsalazine dose/day 1 g | 2 g | 3 g | 4 g |
|---|---|---|---|---|---|
| *Serum:* | | | | | |
| Olsalazine mmol/1 | $C_1$-$C_3$ | 0.010(n=5) | 0.016(n=22) | 0.021(n=16) | 0.026(n=16) |
| | $C_0$ | 0.009(n=7) | 0.017(n=6) | 0.022(n=8) | 0.024(n=10) |
| *Fecal dialysate:* | | | | | |
| 5-ASA mmol/1 | $C_1$-$C_3$ | 6.0 (n=4) | 7.8 (n=18) | 12 (n=12) | 22 (n=12) |
| | $C_0$ | 4.3 (n=5) | 9.5 (n=5) | 17 (n=3) | 25 (n=9) |
| Ac-5-ASA mmol/1 | $C_1$-$C_3$ | 6.9 (n=4) | 8.1 (n=18) | 7.2 (n=12) | 9.7 (n=12) |
| | $C_0$ | 12 (n=5) | 16 (n=5) | 11 (n=3) | 12 (n=9) |

5-ASA = 5-aminosalicylic acid; Ac-5-ASA = acetyl-5-aminosalicylic acid.

Table 9 Concentrations of olsalazine, 5-ASA, and Ac-5-ASA in serum of patients with ulcerative colitis: average in each patient for each dosage of olsalazine

| | | Olsalazine dose/day | | | |
|---|---|---|---|---|---|
| | | 1 g (n=9) | 2 g (n=14) | 3 g (n=20) | 4 g (n=20) |
| Olsalazine | umol/l | 9.2±3.4 | 16±6.8 | 22±8.1 | 25±11 |
| | | (5.4-15) | (3.8-32) | (7.9-39) | (4.5-48) |
| 5-ASA | umol/l | 0.5±0.2 | 1.1±0.9 | 2.4±1.6 | 3.2±2.4 |
| | | (<0.3-1.0) | (<0.3-3.8) | (<0.3-6.8) | (<0.3-8.8) |
| Ac-5-ASA | umol/l | 2.3±1.3 | 3.9±2.4 | 5.7±2.9 | 5.7±3.2 |
| | | (<0.3-4.0) | (<0.3-9.6) | (1.8-14) | (<0.3-12) |

5-ASA = 5-aminosalicylic acid; Ac-5-ASA = acetyl-5-aminosalicylic acid. Values represent means±SD (range).

Table 10 *Concentrations of $PGE_2$ in fecal and rectal dialysates*

| Patients | Study day | Fecal $PGE_2$ levels[a] (ng/ml) | Rectal $PGE_2$ levels[b] (ng/ml) |
|---|---|---|---|
| Ulcerative colitis | | | |
| (SAZ refractory, n=23) | Entry | 1.65±1.80 (0.07-4.48) | 16.0±15.3 (0.29-58.5) |
| | Month 6 | 0.44±1.03# (0.05-4.72) | 8.1±12.3# (0.05-42.8) |
| | Month 12 | 0.77±1.09# (0.06-3.73) | 13.6±15.4 (0.12-49.0) |
| (SAZ intolerant, n=8) | Entry | 1.09±1.18 (0.10-3.18) | 8.5±15.0 (0.31-42.0) |
| | Month 6 | 0.48±0.85 (0.04-2.55) | 5.7±11.9 (0.15-32.6) |
| | Month 12 | 0.16±0.05# (0.08-0.22) | 1.6±2.0 (0.32-5.3) |
| Crohn's colitis (n=9) | Entry | 1.76±1.94 (0.19-4.79) | 4.0±5.1 (0.3-16.3) |
| | Month 6 | 0.70±0.68 (0.08 1.81) | 5.0±4.8 (0.4-13.0) |
| | Month 12 | 1.23±2.06 (0.04-5.39) | 3.0±5.7 (0.1-14.6) |

Values represent mean±SD (range). [a]Normal range: <0.5 ng/ml.
[b]Normal range: <0.6 ng/ml. #Statistically significant ($p<0.05$).

Table 11 Selected characteristics of 23 patients with inactive ulcerative colitis

| | Recruited | Randomized[a] | | Non-randomized |
|---|---|---|---|---|
| | (n=23) | SAZ(n=6) | Placebo(n=5) | (n=12) |
| Age, median year (range) | 48(18-80) | 60(37-65) | 48(26-72) | 37(18-80) |
| Sex, male/female | 11/12 | 4/2 | 2/3 | 5/7 |
| Duration disease, median month (range) | 72(18-480) | 84(36-480) | 108(24-192) | 66(18-228) |
| Duration remission, median month (range) | 16(12-72) | 15(12-24) | 22(12-72) | 12(12-72) |
| Duration of SAZ treatment, median month (range) | 60(12-144) | 48(20-120) | 60(24-144) | 57(12-132) |
| Previously treated with corticosteroids, no. | 23 | 6 | 5 | 12 |
| Smokers/non-smokers, no. | 4/19 | 3/3 | 1/4 | 0/12 |
| Extension of disease[b], 'total'/left sided/ rectal, no. | 6/10/7 | 0/4/2 | 3/1/1 | 3/5/4 |

[a]Randomized because of increased fecal and/or rectal concentrations of $PGE_2$ (Table 12).

[b]Based on previous barium enema. See text for details.

Table 12 Fecal and rectal $PGE_2$ levels, randomization, and outcome in 23 patients with inactive ulcerative colitis in whom sulfasalazine was stopped at entry

| Patient no. | Duration remission (months) | Fecal $PGE_2$/Rectal $PGE_2$ levels (ng/ml) Entry | 2 weeks | 2 months | 6 months | 12 months | 6 months after R[a] | R code | Outcome Remission | Relapse (day) |
|---|---|---|---|---|---|---|---|---|---|---|
| 1 | 12 | 0.34/8.41 | (ND[b]/13.3)[e] | - | - | - | - | - | - | + (14) |
| 2 | 12 | 0.14/0.99 | 0.31/0.33 | 0.14/1.52R | - | - | 0.22/0.26 | SAZ[c] | + | - |
| 3 | 12 | 0.91/26.1 | (0.70/7.32)[e] | - | - | - | - | - | - | + (14) |
| 4 | 72 | 0.73/3.26 | 1.24/3.16R | - | - | - | - | PL[d] | - | + (R+12) |
| 5 | 16 | 0.08/0.49 | 0.20/0.86 | 0.12/0.31 | 0.17/0.82 | 0.25/1.66R | (0.38/2.08)[e] | PL | - | + (R+180) |
| 6 | 17 | 0.08/0.20 | 0.14/0.15 | 0.10/0.46 | 0.42/0.71 | - | - | - | - | + (295) |
| 7 | 54 | 0.20/0.41 | 0.14/0.49 | 0.03/0.19 | 0.28/0.67 | 0.46/0.19 | - | - | + | - |
| 8 | 22 | 0.26/0.30 | 0.16/0.23 | 0.47/0.76 | 3.32/1.54R | - | (8.94/10.3)[e] | PL | - | + (R+159) |
| 9 | 48 | 0.12/0.56 | 0.10/0.24 | 0.37/0.88 | - | - | - | - | - | + (162) |
| 10 | 24 | 0.08/0.13 | 0.12/0.68 | 0.27/0.84 | 0.45/0.30 | 0.14/2.09R | 0.20/0.64 | SAZ | + | - |
| 11 | 72 | 0.09/0.12 | 0.05/0.10 | 0.24/0.29 | 0.12/0.30 | (0.19/9.89)[e] | - | - | - | + (358) |
| 12 | 12 | 0.24/1.26 | 0.24/0.46 | (0.19/23.1)[e] | - | - | - | - | - | + (56) |
| 13 | 12 | ND/0.47 | 0.23/0.31 | 0.30/0.95 | - | - | - | - | - | + (81) |
| 14 | 18 | 0.08/2.13 | 0.32/2.40R | - | - | - | 0.38/0.59 | SAZ | + | - |
| 15 | 12 | 0.50/1.39 | 0.58/2.56R | - | - | - | 0.57/0.23 | PL | + | - |
| 16 | 12 | 0.20/0.55 | 0.32/0.24 | (9.61/ND)[e] | - | - | - | - | - | + (30) |
| 17 | 24 | 1.00/3.59 | 0.99/12.1R | - | - | - | 0.43/2.11 | SAZ | + | - |
| 18 | 12 | 0.37/0.50 | 0.42/0.22 | 0.34/0.31 | - | - | - | - | - | + (156) |
| 19 | 12 | 0.33/0.60 | 0.40/0.32 | 0.35/0.40 | 0.68/1.21R | - | 0.14/ND | SAZ | + | - |
| 20 | 12 | 0.36/1.89 | 0.38/2.49R | - | - | - | 0.32/0.30 | SAZ | + | - |
| 21 | 24 | 0.22/0.46 | 0.18/0.34 | 0.16/0.35 | 0.30/0.27 | 0.27/0.55 | - | - | + | - |
| 22 | 24 | 0.60/3.47 | 2.36/1.42R | - | - | - | (5.38/1.24)[e] | PL | - | + (R+180) |
| 23 | 12 | 0.65/2.21 | 0.30/0.99 | 0.31/0.99 | - | - | - | - | - | + (150) |

[a]R, randomization (i.e., in case of fecal $PGE_2$ >0.5 ng/ml and/or rectal $PGE_2$ >1.0 ng/ml). [b]ND, not done. [c]SAZ, sulfasalazine. [d]PL, placebo. [e]Values in squared brackets indicate that endoscopic relapse was present at the study day. If a relapse occurred the patient was withdrawn.

## Claims

1. The use of in vitro measuring the contents of prostaglandin $E_2$ in samples obtained by means of in vivo dialysis in the rectum of patients with chronic inflammatory bowel disease (IBD), consisting in ulcerative colitis (UC) or Crohn's colitis (CC) for evaluating the disease activity and thereby predicting (a) the outcome of medical treatment in case of serious, active disease, and (b) the risk of relapse in symptom-free patients, who wish to stop the use of relapse preventing medication.

2. The use in vitro measuring according to Claim 1, by which significantly higher prostaglandin $E_2$ pretreatment concentrations are measured compared with the concentrations measured in patients who are known to react on medical treatment, thereby enabling prescription of non-medical treatment, preferably surgical treatment.

3. The use of in vitro measuring according to Claim 1, by which an increase in the luminal concentrations of prostaglandin $E_2$ is measured in patients with inactive UC, thereby enabling prescription of continued medical treatment.

4. The use of in vitro measuring according to Claims 1, 2, or 3, by which measuring of rectal concentrations of prostaglandin $E_2$ in performed on the contents of a dialysis bag, which has been placed in the emptied rectum of the patient.

5. The use of in vitro measuring according to Claim 4, characterized by measuring the concentration of prostaglandin $E_2$ by a radioimmunological method.

## Patentansprüche

1. Verwendung einer in vitro Bestimmung des Gehalts von Prostaglandin $E_2$ in Proben, die mittels in vivo Dialyse im Rektum von Patienten mit einer chronischen, entzündlichen Darmkrankheit (IBD), bestehend in Colitis ulcerosa (UC) oder Crohn'scher Colitis (CC), erhalten wurden, um die Krankheitsaktivität zu ermitteln und dadurch (a) das Ergebnis einer medikamentösen Behandlung im Falle einer ernsten, aktiven Krankheit und (b) das Rückfallrisiko bei symptomfreien Patienten, die das Ende der Anwendung einer rückfallverhindernden Medikamententherapie wünschen, vorherzusagen.

2. Verwendung einer in vitro Bestimmung nach Anspruch 1, durch die signifikant höhere Prostaglandin $E_2$ Vorbehandlungskonzentrationen gemessen werden, im Vergleich zu den Konzentrationen, die bei Patienten gemessen werden, von denen bekannt ist, daß sie auf medikamentöse Behandlung reagieren, wodurch die Verordnung einer nicht medikamentösen Behandlung, vorzugsweise einer chirurgischen Behandlung, ermöglicht wird.

3. Verwendung einer in vitro Bestimmung nach Anspruch 1, durch die ein Ansteig in den luminalen Konzentrationen von Prostaglandin $E_2$ bei Patienten mit inaktiver UC gemessen wird, wodurch eine Verordnung fortgesetzter medikamentöser Behandlung ermöglicht wird.

4. Verwendung einer in vitro Bestimmung nach Anspruch 1, 2 oder 3, wobei die Messung rektaler Konzentrationen von Prostaglandin $E_2$ am Inhalt eines Dialysesacks durchgeführt wird, der in das geleerte Rektum des Patienten gebracht worden ist.

5. Verwendung einer in vitro Bestimmung nach Anspruch 4, gekennzeichnet durch die Messung der Konzentration von Prostaglandin $E_2$ durch eine radioimmunologische Methode.

## Revendications

1. Utilisation de mesure in vitro des teneurs en prostaglandine $E_2$ dans des échantillons obtenus par dialyse in vivo dans le rectum de malades atteints d'une maladie de l'intestin inflammatoire (MII) chronique, consistant en colite ulcéreuse (CU) ou en colite de Chrohn (CC) pour évaluer l'activité de la maladie et prédire de cette manière (a) les résultats d'un traitement médical en cas de maladie sérieuse, active, et (b) le risque de récidive chez des malades asymptomatiques, qui souhaitent cesser l'utilisation d'une médication pour empêcher la récidive.

2. Utilisation de mesure in vitro selon la revendication 1, par laquelle les concentrations de prétraitement en prostaglandine $E_2$ significativement plus élevées, comparées aux concentrations mesurées chez des malades connus pour réagir au traitement médical, sont mesurées, permettant ainsi la prescription d'un traitement non médical, de préférence d'un traitement chirurgical.

3. Utilisation de mesure in vitro selon la revendication 1, par laquelle une augmentation de concentrations luminales en prostaglandine $E_2$ est mesurée chez les malades atteints d'une CU inactive, permettant ainsi la prescription d'un traitement médical poursuivi.

4. Utilisation de mesure in vitro selon les revendications 1, 2, ou 3, par laquelle la mesure de concentrations rectales en prostaglandine $E_2$ est effectuée à partir des teneurs d'un sac de dialyse, qui à été placé dans le rectum vidé du malade.

5. Utilisation de mesure in vitro selon la revendication 4, caractérisée par la mesure de la concentration en prostaglandine $E_2$ par un procédé radioimunologique.

Fig. 1. Concentrations of $PGE_2$ in the rectal lumen of patients with ulcerative colitis correlated to clinical disease activity (semiquantitative grading: $C_0$, $C_1$, $C_{2-3}$), endoscopic disease activity (semiquantitative grading: $E_0$, $E_1$, $E_2$, $E_3$), and histologic disease activity (semiquantitative grading: $H_0$, $H_1$, $H_2$, $H_3$). Values represent medians and $Q_{50}$ interquartile ranges.

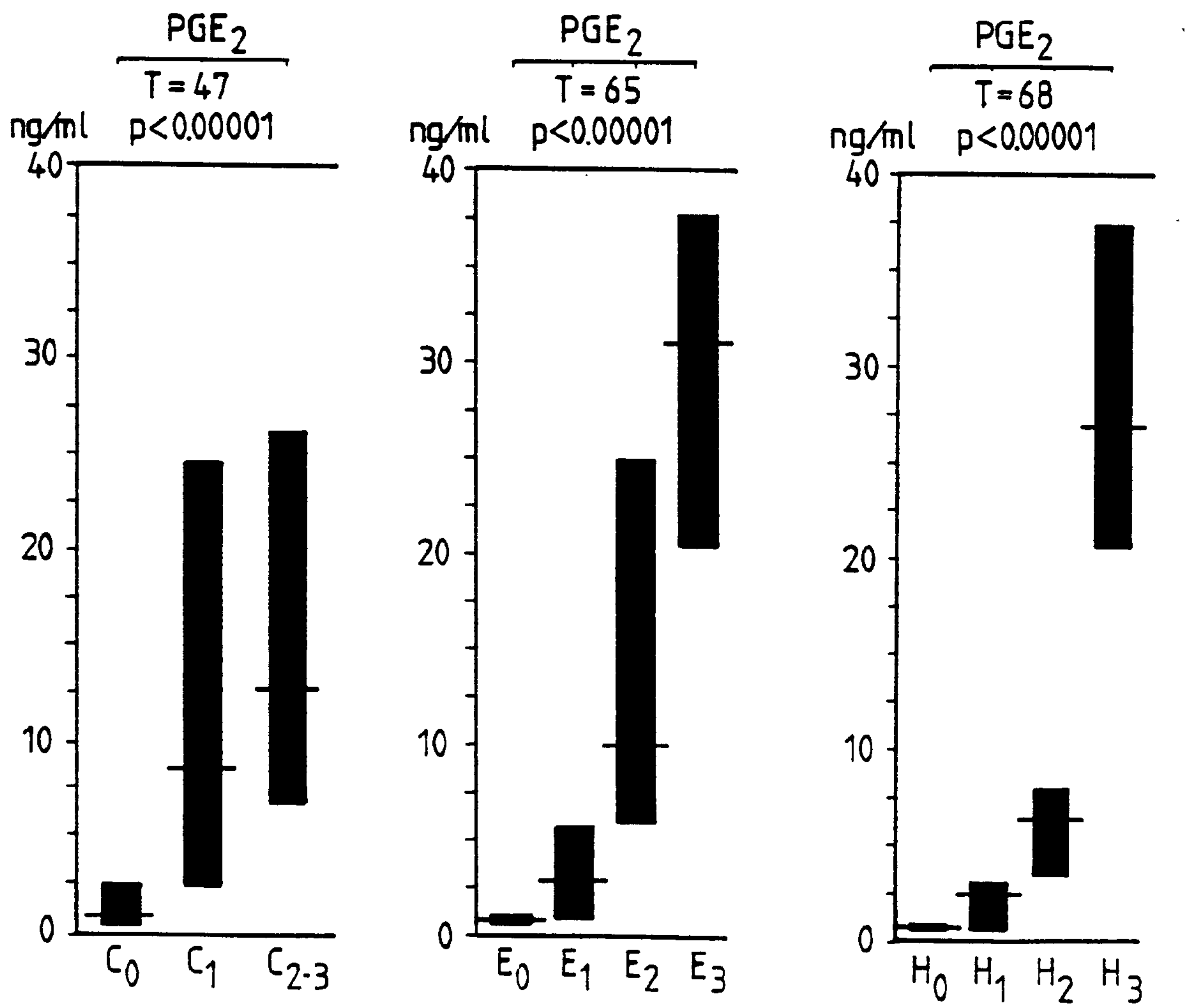

Fig. 2. Concentrations of $PGE_2$ (logarithmic scale) in the rectal lumen of untreated (PRE) patients (n=13) with relapsing ulcerative colitis and after treatment (POST) with Pentasa (5-aminosalicylic acid) enemas (1 g/day) for 2 wk (...) or 4 wk (---). Symbols denote individual outcome of treatment: (●) clinical and endoscopic remission; (o) clinical but not endoscoic remission; (△) unchanged or worse.

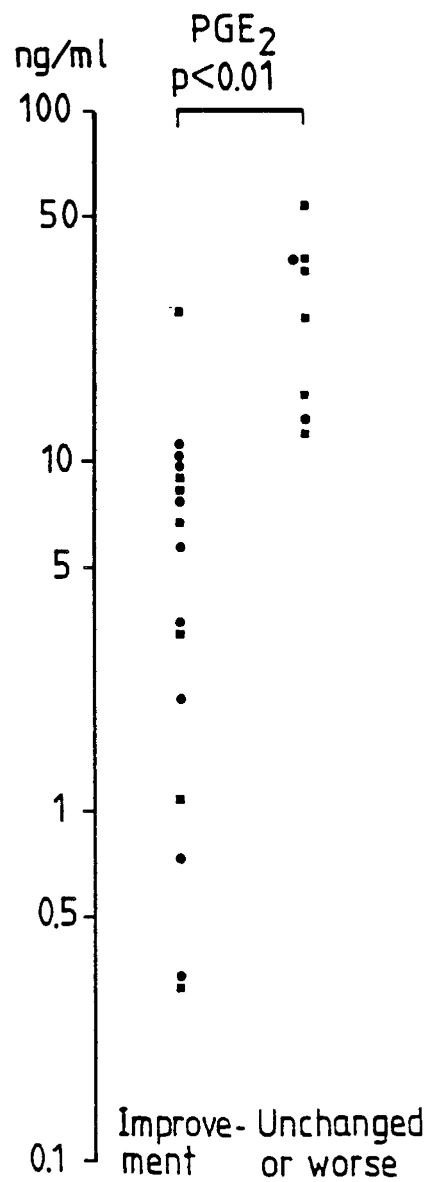

Fig. 3. Concentrations of $PGE_2$ (logarithmic scale) in the rectal lumen of untreated (PRE) patients (n=11) with relapsing ulcerative colitis and efter treatment (POST) with prednisolone enemas (25 mg/day) for 2 wk (...) or 4 wk (--). Symbols denote individual outcome of treatment: (●) clinical and endoscopic remission; (o) clinical but not endoscopic remission; (△) unchanged or worse.

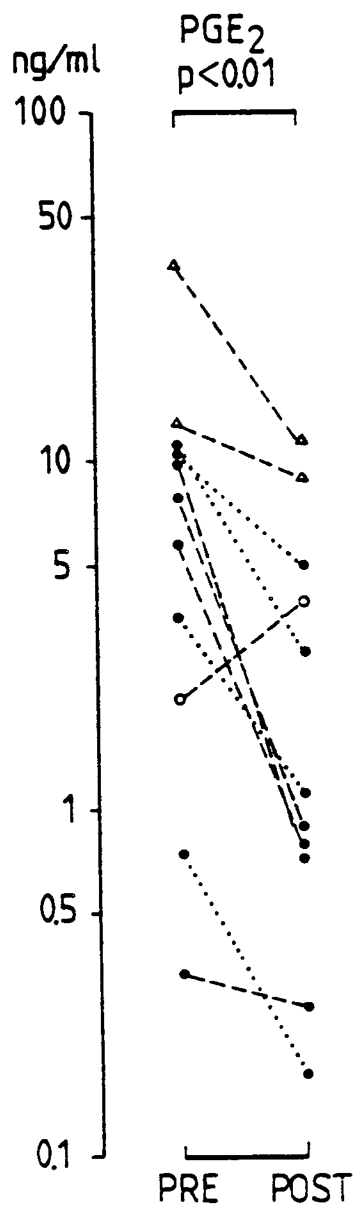

Fig. 4. Pretreatment concentrations of $PGE_2$ and $LTB_4$ (logarithmic scale) in the rectal lumen of patients with relapsing ulcerative colitis correlated to the outcome of medical treatment with pentasa (5-aminosalicylic acid) enemas (1 g/day) (▪) or prednisolone enemas (25 mg/day) (●) for 2-4 wk (n=24).